# EUROPEAN PATENT SPECIFICATION

(11) **EP 0 617 697 B1**
(45) Date of publication and mention of the grant of the patent: **14.08.1996**
(21) Application number: 93900316.6
(22) Date of filing: 18.12.1992
(51) Int. Cl.: C01B 15/037, C11D 3/39

(54) **ALKALINE HYDROGEN PEROXIDE FORMULATION**
ALKALISCHE WASSERSTOFFOXYDFORMULIERUNG
FORMULATION D'EAU OXYGENEE ALCALINE

(30) Priority: 21.12.1991 GB 9127193; 21.01.1992 GB 9201206
(43) Date of publication of application: 05.10.1994
(73) Proprietor: SOLVAY INTEROX LIMITED, Warrington, Cheshire WA4 6HB (GB); JEYES LIMITED, Norfolk IP24 1HA (GB)
(72) Inventor: FEASEY, Neil, David, Widnes Cheshire WA8 7PJ (GB); HEALEY, Brian, James, Norfolk NR16 (GB); MORRIS, Gareth, Wilmot, Wirral Merseyside L63 9YG (GB)
(74) Representative: Pearce, Timothy
(86) International application number: GB9202365
(87) International publication number: WO9313012

(56) References cited:
- EP-A- 0 119 920
- EP-A- 0 188 025
- EP-A- 0 426 949
- WO-A-91/09807
- WO-A-92/06036
- GB-A- 2 072 643

## Description

The present invention relates to aqueous alkaline hydrogen peroxide formulations, and more particularly to a stabilised and buffered formulation.

Households, for many years have employed a general purpose bleach and disinfectant for cleaning and disinfecting hard surfaces such as floors, work surfaces or walls, and especially for disinfecting sanitaryware and waste pipework. One class of formulation which has been especially popular is based on aqueous alkaline hypochlorite solutions. Such formulations have been very effective, but the general public has become aware of academic studies which have shown that in certain circumstances, chlorinaceous compounds can interact with the organic compounds in the environment with the generation of carcinogens or other noxious substances. In consequence, a need has arisen to develop substitute products to offer a "green" alternative for those who wish to avoid contacting hypochlorite with domestic waste or introducing it into effluents that will eventually pass into the municipal sewage treatment works.

Hydrogen peroxide is a product which is generally acceptable environmentally, because its decomposition products are oxygen and water. It has been supplied to the household market in acidic formulations, but it would be preferable to employ alkaline formulations in order to enhance the performance of hydrogen peroxide. However, its employment in the past in aqueous alkaline media has been hindered because alkaline peroxide solutions are prone to decomposition during storage, such as occurs inevitably during the period of distribution from the manufacturer to sales outlets in supermarkets or the like, and storage on their shelves and subsequently in the home of the consumer. The net result of excessive decomposition is that the product progressively loses its efficacy and thereby its shelf life is curtailed. The stability of aqueous alkaline hydrogen peroxide solutions has been improved in GB 2 072 643 and EP-B-00 76 166, both to Interox Chemicals Limited, by employing an aminomethylene phosphonic acid together with a low weight alcohol such as ethanol or isopropanol as a stabiliser system, but the presence of the alcohol inevitably reduces the flash point of the composition and introduces additional processing costs. The incorporation of certain aminomethylene phosphonic acids as stabiliser in alkaline hydrogen peroxide solutions has also been described in International Application WO 91/09807 also to Interox Chemicals Limited.

During the transportation and storage of alkaline hydrogen peroxide solutions, there is also a tendency for a downwards drift of its pH to occur. This can be counteracted to some extent by the incorporation of a buffer appropriate for the desired pH range. It would be convenient to incorporate a buffer in aqueous hydrogen peroxide solutions that is encompassed within the broad descriptive term borates, because a mildly alkaline pH can thereby be maintained longer, but processing difficulties have been encountered in the incorporation of certain borates.

The incorporation of certain borate compounds together with a silicate as stabiliser in short-term alkaline hydrogen peroxide bleaching baths has been described in EP-B-0 119 920 to Atochem, but such solutions are not suitable for distribution and sale to the general public, because they have a much restricted shelf life.

It is an object of the present invention to provide an alkaline hydrogen peroxide solution which overcomes ameliorates one or more of the foregoing difficulties.

According to the present invention, there is provided a storage stable composition suitable for use domestically as a general purpose household bleach and disinfectant which consists essentially of an aqueous alkaline solution of from about 1 to about 10% w/w hydrogen peroxide, at least 0.2% w/w of a borate buffer introduced as sodium tetraborate decahydrate, alkali metal hydroxide in an amount which is sufficient together with the borate buffer to generate a pH in the range of from at least 7 to about 9.5, cyclohexane-1,2-diaminotetrakismethylene phosphonic acid or water soluble salt thereof in a concentration calculated as the acid of at least 150 ppm and optionally a surfactant.

Advantageously, a formulation according to the present invention combines the benefits of stabilising the hydrogen peroxide composition with a most effective compound, cyclohexane-1,2-diaminotetrakis phosphonic acid (CDTMPA) with the practical benefits obtained by introducing disodium tetraborate decahydrate as a borate buffer. The use of the selected buffer prevents the pH of the composition from dropping quickly during its shelf life, ie the time between manufacture and use. Thus, the two components, cooperate to maintain both peroxide and pH stability, and thereby enable the composition to better retain its efficacy during its inevitable shelf-life. Moreover, the use of the selected buffer enables rapid formation of the formulation as a fully dissolved aqueous mixture, thereby minimising the size of plant required for each unit of manufacture and thus minimise processing costs.

In practice, it is preferable to select the concentration of hydrogen peroxide within the range of from about 4% to about 7% by weight. Most conveniently, it can be introduced into the composition in the form of a commercially available aqueous acidic concentrate, which typically contains from about 30 to about 70% w/w hydrogen peroxide. Such solutions typically contain a small concentration of a stabiliser which is adequate for acidic conditions, but which would be inadequate in the buffered alkaline conditions of the instant invention.

The concentration of disodium tetraborate decahydrate buffer in practice is often selected in the range of from about 0.3% to about 1% w/w. Such a range balances the desire to buffer effectively without employing excessive material. In a number of desirable embodiments, its concentration falls in the range of from about 0.4 to about 0.6% w/w.

The alkali metal hydroxide can conveniently comprise sodium hydroxide, in view of its widespread availability and cost. It is often desirable to introduce it into the formulation in the form of a concentrated aqueous solution, such as a solution of from 30 to about 47% w/w.

The stabiliser is most widely available in the acid form, but if desired, it can alternatively be introduced as an alkali metal salt, an example of a water soluble salt. In practice, its concentration in the formulation is usually not greater than about 1500 ppm, and in many suitable formulations, the concentration falls in the range of from 200 to 1000 ppm. In selecting the concentration of added stabiliser, CDTMPA to incorporate, the pH of the buffered composition is preferably taken into account , the higher the pH, the higher the concentration of stabiliser. For a borate buffered composition having a target pH of about pH 8.5, ie +/-about 0.2 pH units, a convenient concentration range of CDTMPA therein is from about 250 to about 500 ppm.

The invention formulation in many embodiments contains a small concentration of a surfactant, such as at least 0.25% by weight, which acts to improve the rate and extent of wetting of the surface which will be disinfected by the formulation. It is desirable to select a nonionic surfactant and especially an amine oxide. Readily available amine oxides can be obtained containing a total of from about 10 to about 20 carbons, of which at least one alkyl chain contains at least 8 linear carbons. Some particularly suitable amine oxides comprise a dimethylalkylamine oxides in which the alkyl group is linear C10 to C16 or mixtures thereof such as those derived from natural sources. The concentration of surfactant is often less than about 3% w/w, and in many practical embodiments is selected in the range of from about 0.4 to about 0.8% w/w. The surfactant can conveniently be introduced into the formulation in the form of an aqueous solution, for example one containing from 15 to 50% w/w active material.

In addition, it is preferable to incorporate within the formulation a perfume which is stable to oxidation in alkaline media, such as in a concentration of from about 0.03 to about 0.15% w/w. It is often convenient to premix the perfume with the surfactant before their introduction into the formulation.

It will be recognised that the invention compositions described herein are essentially free from alcohols or concentrations of other components which would adversely affect their flash point to any significant extent.

The formulations of the present invention can be produced in a number of different sequences of mixing the components together. In general, it is desirable to operate at or nears ambient temperature, ie from about 10 to 40°C, to avoid undue heating costs and avoiding the acceleration of hydrogen peroxide decomposition that could occur at more elevated temperatures.

It is preferable to ensure that the stabiliser is present in the mixture at the time that the peroxide is introduced, such as by introducing the stabiliser before the peroxide is added. It is also desirable to add any alkali as the final step in the process in order to attain a predetermined pH.

A preferred production sequence comprises:-
i dissolving sodium tetraborate decahydrate in a precalculated quantity of water from which alkaline earth metal ions have been removed;
ii introducing the aminomethylene phosphonic acid;
iii blending in an aqueous mixture of the surfactant and the perfume;
iv mixing in aqueous hydrogen peroxide solution, containing nominally 30 to 35% w/w;
v mixing concentrated sodium hydroxide, nominally 47% w/w up to a preset pH.

Advantageously, the stabilised and buffered alkaline hydrogen peroxide formulations of the present invention are able to combine the benefits of improved performance accruing from operation in alkaline conditions compared with operation in acidic conditions with the ability to retain their efficacy for a considerable period of time, both as a disinfectant and as a stain remover from cloth or ceramic surfaces,

Having described the invention in general terms, specific embodiments will now be described in greater detail, by way of example only.

### Example 1

A buffered aqueous alkaline bleach composition was prepared by dissolving 5 parts by weight of disodium tetraborate decahydrate in 825 parts by weight of demineralised water at ambient temperature. 1 Part by weight of a solution containing 30% w/w of cyclohexane-1,2-diaminotetrakismethylene phosphonic acid (the stabiliser) was then introduced with stirring into the clear solution, followed shortly after by an aqueous solution containing 6.25 parts by weight of tetradecyldimethylamine oxide and 0.9 parts by weight of a perfume that is resistant to oxidation which had been made up with water to a total of 25.9 parts by weight. Then, 143 parts by weight of an aqueous hydrogen peroxide solution containing nominally 35% H₂O₂ was mixed into the stabiliser-containing mixture and finally an aqueous solution of sodium hydroxide (nominally 47% w/w) was introduced gradually until the composition had a measured pH of 8.5.

The composition was stored in the dark at either ambient temperature, or 28 or 32°C in a number of bottles which contained a vented closure. The concentration of hydrogen peroxide was monitored at regular intervals employing a standard potassium permanganate titration technique and the extent of hydrogen peroxide decomposition during storage was calculated by comparing the measured concentrations before and after storage. The results are summarised in the Table below.

**The Table**

| Storage Period (weeks) | Storage Temperature | Measured H₂O₂ conc | Loss of H₂O₂ (%) |
|---|---|---|---|
| initial | ambient | 5 0 | - |
| 6 | ambient | 4.91 | 0 |
| 12 | ambient | 5.15 | 0 |
| initial | 28°C | 4.96 | - |
| 6 | 28°C | 5.08 | 0 |
| 12 | 28°C | 4.84 | 1.2 |
| initial | 32°C | 4.91 | - |
| 6 | 32°C | 4.85 | 1.0 |
| 12 | 32°C | 4.71 | 3.9 |

The pH of the compositions were also measured and were still at about pH 8.14 after 12 weeks.

From the Table above, it can be seen that the formulation according to the present invention still contained substantially the same concentration of hydrogen peroxide at the end of the storage period as at the beginning, the maximum loss still being less than 4%, so that the efficacy of the composition remains substantially intact.

### Examples 2 to 4

In these Examples, further compositions were prepared using the same stabiliser and the process described for Example 1 and having the formulations given in Table 2 below. ADMAO represents alkyl dimethyl amine oxide, and NaBD represents sodium borate decahydrate

**Table 2**

| Component | Example 2 | Example 3 | Example 4 |
|---|---|---|---|
| | % w/w | % w/w | % w/w |
| ADMAO - | 0.75 | 0.15 | - |
| | | | |
| Fragrance | 0.08 | 0.04 | - |
| Hydrogen Peroxide | 10.62 | 10.62 | 10.62 |
| Stabiliser | 0.10 | 0.10 | 0.10 |
| NaBD | 0.50 | 0.50 | 0.50 |
| NaOH | to pH 8.5 | to pH 8.5 | to pH 8.5 |
| Soft Water | to 100 | to 100 | to 100 |

## Claims

1. A storage stable composition suitable for use domestically as a general purpose household bleach and disinfectant which consists essentially of an aqueous alkaline solution of from about 1 to about 10% w/w hydrogen peroxide, at least 0.2% w/w of a borate buffer introduced as sodium tetraborate decahydrate, alkali metal hydroxide in an amount which is sufficient together with the borate buffer to generate a pH in the range of from at least 7 to about 9.5, as stabiliser cyclohexane-1,2-diaminotetrakismethylene phosphonic acid or water soluble salt thereof in a concentration calculated as the acid of at least 150 ppm and optionally a surfactant.

2. A composition according to claim 1 in which the concentration of stabiliser is selected in the range of from 200 to 1000 ppm

3. A composition according to either preceding claim in which the concentration of borate buffer is from about 0.4 to about 0.6% w/w calculated as disodium tetraborate decahydrate.

4. A composition according to any preceding claim which has a pH of from about 7.5 to about 8.5.

5. A composition according to any preceding claim which contains at least 0.25% w/w surfactant.

6. A composition according to any preceding claim which contains an amine oxide surfactant.

7. A composition according to claim 6 in which the amine oxide contains a linear C10 to C16 alkyl and two short chain alkyl substituents.

8. A process for producing a stabilised aqueous alkaline solution of hydrogen peroxide in which hydrogen peroxide is introduced to a concentration of at least 3% w/w in the presence of an effective amount of cyclohexane-1,2-diaminotetrakismethylene phosphonic acid or water soluble salt thereof as stabiliser into an aqueous solution of at least 0.2% w/w disodium tetraborate decahydrate, the composition is adjusted to a pH of from 8 to 9.5 where necessary, by the introduction of an alkali metal hydroxide, and at least 1% w/w of a surfactant is introduced, either before or after the hydrogen peroxide.

## Patentansprüche

1. Lagerbeständige, zur Verwendung als Allzweck-Haushalts-bleich- und Desinfektionsmittel im Haushalt geeignete Zusammensetzung, welche im wesentlichen aus einer wässrigen alkalischen Lösung von ca. 1 bis ca. 10 Gew.-% Wasserstoffperoxid, mindestens 0,2 Gew.-% eines als Natriumtetraboratdecahydrat eingeführten Boratpuffers, Alkalimetallhydroxyd in einer Menge, welche zusammen mit dem Boratpuffer genügend groß ist, um zu einem pH-Wert im Bereich von mindestens 7 bis ca. 9,5 zu führen, Cyclohexan-1,2-diaminotetrakismethylenphosphonsäure oder dem wasserlöslichen Salz derselben in einer Konzentration von mindestens 150 ppm, auf die Säure bezogen, als Stabilisator und gegebenenfalls einem Tensid besteht.

2. zusanmensetzung nach Anspruch 1, wobei die Konzentration des Stabilisators im Bereich von 200 bis 1000 ppm gewählt wird.

3. Zusammensetzung nach einem der vorhergehenden Ansprüche, wobei die Konzentration des Boratpufers zwischen ca. 0,4 und ca. 0,6 Gew.-%, auf das Dinatriumtetraboratdecahydrat bezogen, liegt.

4. Zusammensetzung nach einem der vorhergehenden Anspüche, welche einen pH-Wert von ca. 7,5 bis 8,5 aufweist.

5. Zusammensetzung nach einem der vorhergehenden Ansprüche, welche mindestens 0,25 Gew.-% Tensid enthält.

6. Zusammensetzung nach einem der vorhergehenden Ansprüche, welche ein Aniinoxidtensid enthält.

7. Zusammensetzung nach Anspruch 6, wobei das Aminoxid ein lineares C10- bis C16-Alkyl und zwei kurzkettige Aklylsubstituenten enthält.

8. Verfahren zur Herstellung einer stabilisierten wässrigen alkalischen Lösung von Wasserstoffperoxid, wobei das Wasserstoffperoxid zur Erzielung einer Konzentration von mindestens 3 Gew.-% in Gegenwart einer wirksamen Menge Cyclohexan-1,2-diaminotetrakismethylenphosphonsäure oder des wasserlöslichen Salzes derselben als Stabilisator in eine wässrige Lösung von mindestens 0,2 Gew.-% Dinatriumtetraboratdecahydrat eingeführt wird, wobei die Zusammensetzung gegebenenfalls durch die Einführung eines Akalimetallhydroxids auf einen pH-Wert von 8 bis 9,5 eingestellt und mindestens 1 Gew.-% eines Tensids entweder vor oder nach dem Wasserstoffperoxid eingeführt wird.

## Revendications

1. Une composition stable à la conservation convenant à l'usage en milieu ménager comme produit de blanchiment et désinfectant universel qui comporte essentiellement une solution aqueuse alcaline de peroxyde d'hydrogène à une concentration comprise entre environ 1% et environ 10% p/p, au moins 0,2% p/p d'un borate tampon introduit sous forme de tétraborate de sodium décahydraté, de l'hydroxyde d'un métal alcalin en quantité suffisante pour générer avec le borate tampon un pH compris entre au moins 7 et environ 9,5, de l'acide cyclohexane-1,2-diaminotétrakisméthyléne phosphonique ou un sel hydrosoluble de cet acide comme stabilisant à une concentration calculée en tant qu'acide d'au moins 150 ppm et facultativement un tensioactif.

2. Une composition selon la revendication 1 où la concentration du stabilisant est choisie dans l'intervalle de 200 à 1000 ppm.

3. Une composition selon l'une ou l'autre de revendications précédentes où la concentration du borate tampon est située entre environ 0,4 et environ 0,6% p/p calculée en tant que tétraborate disodique décahydraté.

4. Une composition selon l'une quelconque des revendications précédentes dont le pH est situé entre environ 7,5 et environ 8,5.

5. Une composition selon l'une quelconque des revendications précédentes, qui contient au moins 0,25% p/p de tensioactif.

6. Une composition selon l'une quelconque des revendications précédentes, qui contient un tensioactif à base d'oxyde d'amine.

7. Une composition selon la revendication 6 dans laquelle l'oxyde d'amine comporte une chaîne alkylique de C10 à C16 et deux substituants alkyliques à chaîne courte.

8. Un procédé pour la production d'une solution alcaline aqueuse stabilisée de peroxyde d'hydrogène où le peroxyde d'hydrogène est introduit jusqu'à une concentration d'au moins 3% p/p en présence d'une quantité efficace d'acide cyclohexane-1,2-diaminotetrakisméthylène phosphonique ou d'un sel hydrosoluble de cet acide comme stabilisant dans une solution aqueuse d'au moins 0,2% p/p de tétraborate disodique décahydraté, le pH de la composition est adjusté lorsque nécessaire à une valeur entre 8 et 9,5 par l'introduction de l'hydroxyde d'un métal alcalin, et au moins 1% p/p d'un tensioactif est introduit, soit avant soit après le peroxyde d'hydrogène.
